# EUROPEAN PATENT APPLICATION

(11) **EP 3 098 739 A1**
(43) Date of publication of application: **30.11.2016**
(21) Application number: 16169765.1
(22) Date of filing: 16.05.2016
(51) Int. Cl.: G06F 19/00

(54) **HEALTHCARE COMMUNICATION SYSTEM**

(30) Priority: 26.05.2015 US 201562166655 P
(71) Applicant: Hill-Rom Services, Inc., Batesville, IN 47006-9167 (US)
(72) Inventor: RIBBLE, David Lance, Indianapolis, IN Indiana 46202 (US); HUSTER, Keith A, Sunman, IN Indiana 47041 (US); LINGENFELSER, Joshua Peter, Fuquay Varina, NC North Carolina 27526 (US); BRASAC, Teresa, Miami Shores, FL Florida 33138 (US); WALTON, Kelly F, Cary, NC North Carolina 27518 (US)
(74) Representative: Findlay, Alice Rosemary

(57) **Abstract**

A hospital information system of a healthcare facility includes a patient safety protocol module of a safety protocol management system that is configured to manage a patient safety protocol for each of a plurality of patients being treated at the healthcare facility. Each of the patient safety protocols, based on health information of a patient and information of one or more care assets available to be assigned to the patient, address a health risk of the patients being treated at the healthcare facility. The patient safety protocol module is further configured to analyze information of patients, including care assets assigned to the patients and outcomes of the patients. The patient safety protocol module is further configured to adjust the patient safety protocol based on the analysis.

## Description

The present disclosure relates generally to healthcare communication systems such as patient-nurse communication systems, and more particularly to implementing safety protocols that include alerts of the healthcare communication system being set based on conditions of a patient.

Healthcare communication systems typically include information and communication technologies to support health services conducted in a healthcare facility setting. For example, some healthcare communication systems include patient-nurse communication systems or "nurse call" systems that facilitate communication among members of a nursing staff and other persons dispersed throughout the healthcare facility. The nurse call systems generally provide information about the present status or condition of patients in the healthcare facility, and may additionally provide information regarding status information pertaining to patient supports (e.g., hospital beds, chairs, lifts, stretchers, etc.) throughout the healthcare facility. Examples of such prior art nurse call systems are Hill-Rom's COMposer™ communication system and Hill-Rom's COMLinx™ communication system.
Typically, a member of the nursing staff sets which alerts the nurse call system will provide based on a safety protocol, such as a fall risk safety protocol assigned to a patient that has been identified as a fall risk, and maintains compliance with the safety protocol. Under the fall risk safety protocol, it may not be desirable for the fall risk patient to get out of bed unless a caregiver is present in the room to assist the fall risk patient. Accordingly, an alert may be set to trigger an alert, or notification, at the nurse call system when the fall risk patient exits a hospital bed, for example. Additionally, sensory indicators (e.g., visual, audible, etc.) are often associated with the nurse call systems to notify the hospital staff of the present state of the alerts, such as whether they are alarmed, or triggered. For example, a sensory indicator may be alarmed to indicate that an alert was violated, such as when the falls risk patient has exited the hospital bed.

The present application discloses one or more of the following features alone or in any combination.

According to one aspect of the present disclosure, a system comprises a patient support apparatus, a user input device, and a safety protocol management system. The patient support apparatus includes a plurality of sensors coupled to a communication network. The user input device is coupled to the communication network. The safety protocol management system is coupled to the communication network. The safety protocol management system includes a patient safety protocol module. The safety protocol management system is configured to receive information from the patient support apparatus and user input device over the communication network and to manage a patient safety protocol for a patient associated with the patient support apparatus to address a safety risk of the patient. Each of the patient safety protocols is based on health information of the patient. The patient safety protocol module is configured to retrieve historical outcomes for other patients that were associated with the patient support apparatus, identify the patient support apparatus as a root cause of a negative patient outcome for the other patients, and adjust a patient safety protocol based on the root cause determination.

In some embodiments, adjusting the patient safety protocol includes identifying an environmental condition that should be modified to reduce the risk of an adverse event occurring for the patient and alerting a caregiver to modify the environmental condition.

In some embodiments, adjusting the patient safety protocol includes identifying a parameter of the patient support apparats that should be modified to reduce the risk of an adverse event occurring for the patient and alerting a caregiver to modify the parameter.

In some embodiments, adjusting the patient safety protocol includes identifying an alternate patient support apparatus that should be used for the patient and alerting a caregiver to substitute the alternate patient support apparatus.

In some embodiments, the system further comprises an electronic medical records system coupled to the communication network and identifying an alternate patient support apparatus includes considering details of the patient's medical record to determine a risk profile of the patient and utilizing the risk profile to identify an appropriate alternate patient support apparatus to reduce the risk profile.

In some embodiments, the system further comprises an asset management system for monitoring the availability of patient support apparatuses and identifying an alternate patient support apparatus includes considering the availability of alternate patient support apparatuses to identify an appropriate alternate patient support apparatus.

In some embodiments, adjusting the patient safety protocol includes identifying an optimal parameter setting of the alternate patient support apparats that should be selected to reduce the risk of an adverse event occurring for the patient and alerting a caregiver to select the optimal parameter on the alternate patient support apparatus.

In some embodiments, adjusting the patient safety protocol includes identifying an optimal environmental setting that should be selected to reduce the risk of an adverse event occurring for the patient and alerting a caregiver to select the optimal environmental setting.

In some embodiments, the patient support apparatus communicates a change in an operating parameter of the patient support apparatus over the communication network and the a safety protocol management system monitors the communication network for changes in parameters of the patient support apparatus to determine a safety risk of the patient.

In some embodiments, a change in an operating parameter of the patient support apparatus is processed by the safety protocol management system to update a risk profile of the patient on the patient support apparatus.

In some embodiments, the system further comprises an electronic medical records system, wherein an input to the user input device indicative of a desired change in an operating parameter of the patient support apparatus is communicated over the communication network, and wherein the input is processed by the electronic medical records system to update the patient's electronic medical record to indicate that the patient has developed a risk factor associated with the change in the changed operating parameter.

In some embodiments, the safety protocol module processes the risk factor associated with the change in operating parameter to re-evaluate the risk of an adverse event to the patient, and, if the risk of an adverse event meets a predetermined threshold, alert a caregiver of the risk.

In some embodiments, an operating parameter of the patient support apparatus includes head angle, an alarm setting, a therapy setting, a component orientation, a mattress pressure, a brake setting, a siderail position, or a motion status.

In some embodiments, an environmental condition includes a location, a temperature level, a lighting condition, a pressure reading, a position setting, or an alert setting.

In some embodiments, the user input device receives information related to the use of a care asset such as an alternative patient support, a chair, a toilet, a lift, a sling, a stretcher, an article of clothing, gloves, socks, a mask, a gown, a pad, a bedding item, a sheet, a blanket, a catheter, a central line kit, a ventilator, a blood pressure monitor, an EKG monitor, an EEG monitor, a SpO₂ monitor, a heart rate monitor, a respiration monitor, a scale, a thermometer, a telemetry system, an infusion system, a sequential compression device, or exercise equipment.

According to another aspect of the present disclosure, a hospital information system for use in a healthcare facility that comprises a safety protocol management system that includes a patient safety protocol module. The safety protocol management system is configured to manage a patient safety protocol for each of a plurality of patients being treated at the healthcare facility. Each of the patient safety protocols address a safety risk of the patients being treated at the healthcare facility. Each of the patient safety protocols is based on health information of a patient and information of one or more care assets available to be assigned to the patient. The patient safety protocol module is configured to retrieve historical outcomes for patients that were treated at the healthcare facility, associate care assets used to treat the patients with the patients treated by the care assets, identify one or more of the associated care assets as a root cause of a negative patient outcome, and adjust the patient safety protocol based on the one or more of the associated care assets identified as the root cause of one or more patients that developed the health risk.

In some embodiments, to identify one or more of the associated care assets as a root cause of a negative patient outcome comprises to determine whether one or more of the associated care assets used to treat the patients at the healthcare facility caused one or more of the patients that were treated by the one or more of the associated care assets to develop a health risk at a rate greater than a threshold rate

In some embodiments, to determine whether other patients developed the health risk comprises to determine a correlation between an outcome for each of the patients treated at the healthcare facility and one or more of the care assets used by each of the patients treated at the healthcare facility.

In some embodiments, the patient safety protocol module is further configured to trigger an evaluation of the patient safety protocol for a patient of the healthcare facility in response to a determination that a correlation exists between one or more of the plurality of care assets presently assigned to the patient that are identified as a health risk to the patient. The patient safety protocol module is further still to adjust the patient safety protocol of the patient based on the one or more of the plurality of care assets identified as the risk.

In some embodiments, the health information includes a medical history of the patient.

In some embodiments, the safety protocol management system is further configured to retrieve the health information from an electronic medical record (EMR) system of the hospital information system.

In some embodiments, the care asset information includes an indication of care assets presently assigned to the patient and an indication of available care assets that are available for being put into use to treat the patient.

In some embodiments, the safety protocol management system is further configured to retrieve the care asset information from a care asset management system of the hospital information system.

In some embodiments, the care asset information includes an identifier, a location, an assigned patient, and a usage history.

In some embodiments, the hospital information system includes a nurse call system to provide one or more alarms corresponding to one or more alerts that have been triggered by an event. The nurse call system includes a display operable to render a graphical user interface (GUI) to interface provides an interface a staff member of the healthcare facility to set the one or more alerts and deactivate the one or more alarms. The event is triggered by one of an action of the patient or a condition of a care asset presently assigned to the patient.

In some embodiments, the care assets include at least one of a patient support, a support pad, a catheter, a central line kit, and a ventilator.

In some embodiments, the patient support comprises one of a chair, a toilet, a stretcher, or a lift.

The invention will now be further described by way of example with reference to the accompanying drawings, in which:
Fig. 1 is a simplified schematic showing a logical architecture for a healthcare communication system that includes a hospital information system in network communication with one or more patient supports;
Fig. 2 is a simplified schematic of the hospital information system of Fig. 1 that includes a safety protocol management system having a safety protocol module;
Fig. 3 is a simplified schematic showing physical components of an embodiment of one of the patient supports of FIG. 1 that includes a number of sensors and interfaces to adjust components of the patient support;
Fig. 4 is a flow diagram of a process for administering a safety protocol that may be executed by the hospital information system of Fig. 1;
Fig. 5 is a flow diagram of a process for monitoring patient outcomes of the safety protocol administered in Fig. 4; and
Fig. 6 is a flow diagram of a process for adjusting the safety protocol administered in Fig. 4 that may be executed by the safety protocol module of Fig. 2.

Aspects of the present invention are described with reference to certain illustrative embodiments shown in the accompanying drawings and described herein.

In general, a healthcare communication system includes one or more staff or nursing computers or computing devices, which may be referred to as stations or consoles. The stations or consoles, in cooperation with various computers, networks, and supporting equipment and services, enable nurses and other staff to receive, view, manage, and route, output, or respond to electrical and wireless signals from a variety of communication, call, monitoring, detecting, and/or signaling devices. Some communication, call, monitoring, detecting, and/or signaling devices are activated by patients, staff, or visitors. Others are activated by the occurrence of an event or alarm condition detected by signal receivers, patient monitoring equipment, or patient supports (e.g., hospital beds) located throughout a healthcare facility, such as from sensors integrated into hospital beds. For example, when the healthcare communication system receives a signal from a communication, call, monitoring, detecting, and/or signaling device, one or more indicator assemblies may be activated to alert hospital staff (e.g., nurses, caregivers, etc.) of the condition or event being signaled by the communication, call, monitoring, detecting, and/or signaling device. Accordingly, the hospital staff may respond based on the alert condition (i.e., an alarmed alert) or event signaled by the communication, call, monitoring, detecting, and/or signaling device. Additionally, the healthcare communication system is configured to log patient data, which the hospital staff may rely upon when treating patients at the healthcare facility.

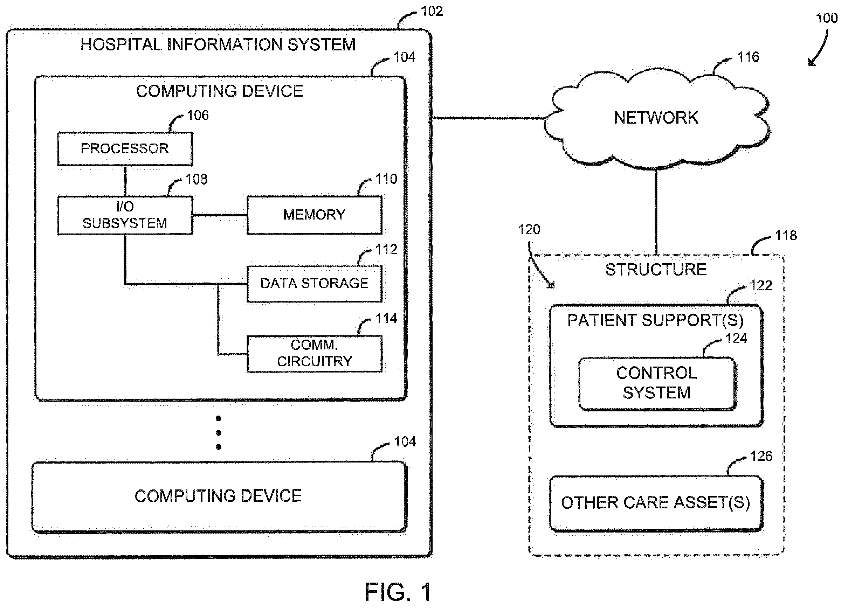

One such embodiment of a healthcare communication system 100 to manage communications throughout the healthcare facility is diagrammatically illustrated in Fig. 1. The healthcare communication system 100 includes a hospital information system 102 of one or more hospitals communicatively coupled via a network 116 to various care assets 120, such as a patient support apparatus 122 and/or other care assets 126 of a structure 118 (e.g., a hospital room, a ward in a hospital, etc.). The care assets 120 may be marked or otherwise electronically recorded as being in various states of availability. For example, the various availability states may include an assigned state indicating that the care asset 120 is assigned to a particular patient, an available state indicating that the care asset 120 is in a condition to be assigned to a patient, an awaiting maintenance state indicating that the care asset 120 cannot be assigned until maintenance is performed, etc. Similar to the patient data being logged at the hospital information system 102, care asset data (e.g., usage data, location data, etc.) of the healthcare communication system 100 can also be logged by the hospital information system 102.

As such, an analysis can be performed at the hospital information system 102 on the patient data and the care asset data. For example, as will be described in further detail below, the care asset data may be compared against patient outcomes that can be used to determine whether a correlation exists between a particular negative patient outcome and one or more care assets that are either presently in use by a patient or available to be assigned to a patient. Accordingly, a present safety protocol can be adjusted based on whether the correlation exists. In other words, the safety protocol implemented for a particular patient can be modified to reduce or altogether eliminate a health risk identified for that patient based on the identified negative patient outcome resulting from the one or more care assets. Further, the modifications to the safety protocol may trigger a care asset 120 affected by the modifications to take measures to ensure the modification is being adhered to, such as by ensuring other care assets 120 are present in accordance with the adjusted safety protocol and/or activating additional/or alternative alerts in accordance with the adjusted safety protocol. Still further, a particular safety protocol may be activated or modified based on a detected use of a particular care asset 120, if that care asset 120 part of the active safety protocol, the usage of which therefore indicates that the patient's status has changed.

The safety protocol includes one or more protocols, such as care protocols, therapy protocols, preventative protocols, etc., that are each associated with a patient care action or care asset 120. Further, each of the protocols of the safety protocol may also include one or more sub-protocols. For example, a patient may be identified as a falls risk and a pressure ulcer risk. Accordingly, the safety protocol may include a falls risk safety protocol and a skin safety protocol for that patient. Further, because the patient is a pressure ulcer risk, which is a particular skin safety concern, a pressure ulcer prevention sub-protocol may be implemented. As such, the care assets 120 associated with the more specific pressure ulcer prevention sub-protocol may be assigned to the patient, rather than the care assets 120 associated with the more broad skin safety protocol, which may include care assets 120 that are not necessary or that may not directly address the pressure ulcer risk. For example, a particular patient support surface/mattress may be sufficient for a skin safety protocol, while a different, more specialized patient support surface/mattress is indicated for a patient with a pressure ulcer risk. Further, settings of the care assets 120 relevant to the pressure ulcer prevention sub-protocol may be changed (e.g., frame controls set to prevent a head-of-bed angle greater than one of 30 or 45 degrees, as defined by the pressure ulcer prevention sub-protocol) and/or alerts relevant to the pressure ulcer prevention sub-protocol may be changed (e.g., a chair pressure pad and chair sensing system set to alert if a patient is on the chair and the pressure reducing chair pad is not in use).

To perform the analysis, as well as to facilitate the transfer of data and other network communications across the hospital information system 102, the hospital information system 102 includes a number of computing devices 104. Each of the computing devices 104 may be embodied as any type of computation or computer device capable of performing the functions described herein, including, without limitation, a server (e.g., stand-alone, rack-mounted, blade, etc.), a network appliance (e.g., physical or virtual), a high-performance computing device, a web appliance, a distributed computing system, a computer, a processor-based system, a multiprocessor system, a smartphone, a tablet computer, a laptop computer, a notebook computer, and/or a mobile computing device. The illustrative computing device 104 of Fig. 1 includes a processor 106, an input/output (I/O) subsystem 108, a memory 110, a data storage device 112, and communication circuitry 114. Of course, the computing device 104 may include additional and/or alternative components, such as those commonly found in a computer (e.g., various input/output devices), in other embodiments. Additionally, in some embodiments, one or more of the illustrative components may be incorporated in, or otherwise form a portion of, another component. For example, the memory 110, or portions thereof, may be incorporated in the processor 106 in some embodiments.

The processor 106 may be embodied as any type of processor capable of performing the functions described herein. For example, the processor 106 may be embodied as a single or multi-core processor(s), digital signal processor, microcontroller, or other processor or processing/controlling circuit. The memory 110 may be embodied as any type of volatile or non-volatile memory or data storage capable of performing the functions described herein. In operation, the memory 110 may store various data and software used during operation of the computing device 104 such as operating systems, applications, programs, libraries, and drivers. The memory 110 is communicatively coupled to the processor 106 via the I/O subsystem 108, which may be embodied as circuitry and/or components to facilitate input/output operations with the processor 106, the memory 110, and other components of the computing device 104. For example, the I/O subsystem 108 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations.

The data storage device 112 may be embodied as any type of device or devices configured for short-term or long-term storage of data such as, for example, memory devices and circuits, memory cards, hard disk drives, solid-state drives, or other data storage devices. In use, as described below, the data storage device 112 and/or the memory 110 may store security monitoring policies, configuration policies, or other, similar data. The communication circuitry 114 may be embodied as any communication circuit, device, or collection thereof, capable of enabling communications between the computing devices 104 and/or between one of the computing devices 104 and the care assets 120. The communication circuitry 114 may be configured to use any one or more communication technology (e.g., wired or wireless communications) and associated protocols (e.g., Ethernet, Bluetooth®, Wi-Fi®, WiMAX, etc.) to effect such communication.

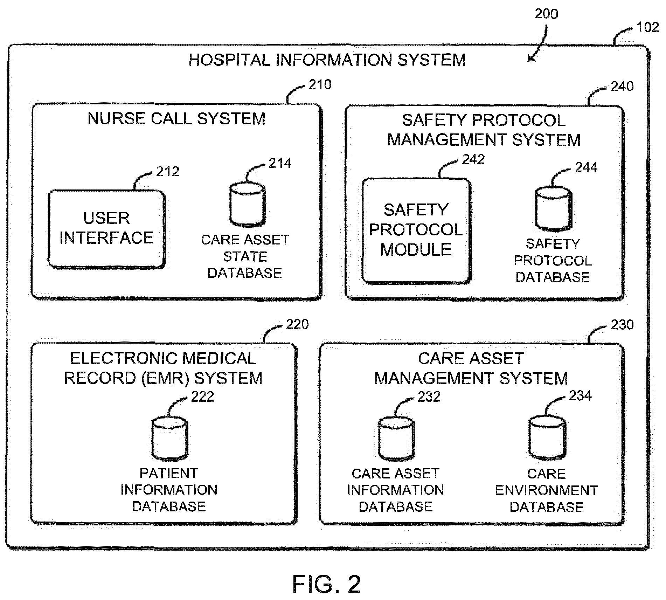

The computing devices 104 of the hospital information system 102 may be configured into separate subsystems for managing data and coordinating communications throughout the hospital information system 102. For example, in an illustrative hospital information system 200 shown in Fig. 2, the hospital information system 200 includes a nurse call system 210, an electronic medical record (EMR) system 220, a care asset management system 230, and a safety protocol management system 240, each of which may be separately or collectively embodied as one or more computing devices 104. It should be appreciated that, in some embodiments, one or more of the subsystems of the illustrative hospital information system 200 may be combined and/or include additional and/or alternative subsystems. For example, in some embodiments, at least a portion of the electronic medical record (EMR) system 220 and the care asset management system 230 may be combined in a single alternative subsystem, such as in an admission, discharge, and transfer (ADT) system.

The nurse call system 210 is configured to operate and manage many of the primary nurse call functions of the hospital information system 102. For example, the nurse call system 210 may be configured to receive and manage messages from various connected devices (e.g., the care assets 120), coordinate assignment of patients to hospital supports, and/or to synchronize connected devices within the healthcare communication system 100 that are in network communication with the nurse call system 210. Additionally or alternatively, the nurse call system 210 may be configured to answer, control placement of, and cancel calls, as well as generate alarms, acknowledge and cancel alarms, and/or manage location information for staff and the care assets 120. Accordingly, the illustrative nurse call system 210 includes a care asset state database 214 in which to store care asset state information. The care asset state information may include any type of data indicative of a present state of a care asset and/or an alert associated with, or triggered by, a care asset (e.g., whether an alert is in an alarmed state).

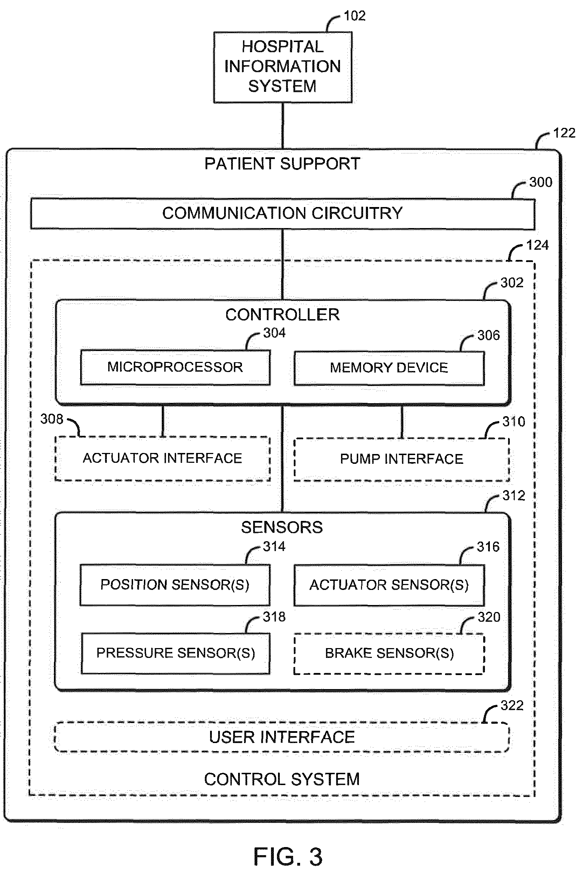

It should be appreciated that the nurse call system 210 is configured to interface with the various care assets 120 at the structure 118, such that the alerts, described in further detail below, may be triggered by the care assets 120 and an indication of such triggered alerts may then be transmitted to the nurse call system 210 for reconciliation. As such, a communication interface (not shown) of the network 116 for facilitating network communications across the network 116 may be present within the structure 118 (e.g., an Ethernet port on a wall in a hospital room of the structure 118, a wireless access point accessible to a ward of the structure 118, etc.). Additionally, the care assets 120 may include a communication interface (see the communication circuitry 300 of Fig. 3), which may be coupled to a bed interface unit (BIU) of the patient support apparatus 122, for example. Additionally or alternatively, the communication interface of the other care assets 126 may include a wireless transceiver (e.g., Bluetooth®, Zigbee®, Wi-Fi®, WiMAX, etc.) configured to wirelessly communicate with another wireless transceiver of a care asset 120 that has also been designated as a central communication hub for a patient (e.g., a hospital bed embodiment of the patient support apparatus 122). Accordingly, in such embodiments, the other care assets 126 may communication with the hospital information system 102 via the intermediary care asset 120 designated as the central communication hub.

To support the interactions between the staff and the nurse call system 210 (i.e., to indicate the state of the alerts for a particular patient and/or care asset 120), the illustrative nurse call system 210 includes a user interface (UI) 212. In some embodiments, the user interface 212 may include a display (not shown) that is operable to generate or display a graphical user interface (GUI) to enable the staff to interface with the nurse call system 210. Additionally or alternatively, in some embodiments, the user interface may include visual indicators (e.g., light emitting diodes (LEDs)) that represent different states of the care assets 120 and/or the alerts representative thereof, and/or audible indications (e.g., via a speaker) that indicate the different states of the care assets 120 via tone and/or spoken language (e.g., via a voice generator). Further, in some embodiments, the nurse call system 210 may be additionally configured to activate/deactivate staff, manage staff-patient assignments, assign and manage roles and responsibilities to staff and devices, as well as manage patient information and patient discharges/transfers.

In use, the EMR system 220 aggregates and stores the medical and clinical data of the healthcare communication system 100 of Fig. 1. Accordingly, the illustrative EMR system 220 includes a patient information database 222 for storing the medical and clinical data of each patient of the hospital information system 102. The medical and clinical data stored at the patient information database 222 may include any type of data relative to a patient and/or an outcome of the patient, such as a history of medications administered and/or prescribed to a patient, care assets 120 assigned to a patient, health history of a patient, etc. It should be appreciated that, in some embodiments, the EMR system 220 may additionally include medical and clinical data for particular patients received from other healthcare communication systems, such as may be included in electronic health records (EHRs).

The care asset management system 230 is configured to manage care assets of the healthcare communication system 100. To do so, in some embodiments, the care asset management system 230 may store information relative to the care assets 120 of the healthcare communication system 100 in a care asset information database 232. The care asset information stored at the care asset information database 232 may include any type of data that is indicative of a state of a care asset 120, such as a component usage history, a maintenance history, an assignment history, an illness exposure history, etc. Additionally, the illustrative care asset management system 230 includes a care environment database 234 in which to store care environment information. The care environment information may include any type of information indicative of the environment of a care asset 120 assigned to a patient, such as a location, a temperature level, a lighting condition, a pressure reading, a position setting, an alert setting, etc.

The safety protocol management system 240 is configured to manage the safety protocols of the healthcare communication system 100. As described previously, each of the safety protocols includes a number of various protocols (e.g., care protocols, therapy protocols, preventative protocols, etc.) and sub-protocols that are typically based on the present conditions of a patient and/or expected conditions of the patient, as well as the care assets 120 assigned to the patient. Accordingly, the illustrative safety protocol management system 240 includes a safety protocol database 244 for storing the safety protocols.

In some embodiments, the safety protocol may be based on a patient scoring system to determine a risk score determined from a set of established risk factors or arise from a doctor's order, for example. Illustratively, the safety protocol includes a set of defined procedures (i.e., instructions) that, when executed, prompt members of the hospital staff to ensure certain precautions are taken. Additionally, the safety protocol includes a set of alerts to be set relative to the presently implemented safety protocol. For example, a falls risk protocol may be implemented for a patient identified as a falls risk. In such an embodiment, the falls risk protocol may activate an alert that will alarm (i.e., provide a notification) when a falls risk patient exits a patient support apparatus 122, such as a hospital bed, a chair, a toilet, etc. In other words, an exit alarm may be activated at one or more of the patient support apparatuses 122 assigned to the patient, or in the hospital room in which the patient is assigned, based on the implemented safety protocol.

Further, in some embodiments, elements of the care environment (e.g., lights, sensors, etc.) may be adjusted based on the safety protocol. Accordingly, the safety protocol may include one or more care environment settings. The care environment settings may include a minimum illumination (e.g., lumens) level, a light (e.g., mounted on the floor, wall, ceiling, hospital bed, etc.) setting, a sensor (e.g., motion detection) setting, and/or the like. Additionally, in some embodiments, a sensory (e.g., audible and/or visible) designation may be provided based on the safety protocol, such as a message on a display component (e.g., on electronic patient signage) placed in a location that is visible by the hospital staff and/or one or more other care assets 126 that can be assigned to the patient based on the safety protocol (e.g., red socks to indicate the patient is a falls risk). Accordingly, in such embodiments wherein the visible designation requires one or more of the other care assets 126, the other care asset 126 may be automatically ordered when the safety protocol is designated for that patient, such as by an instruction from the safety protocol management system 240 (e.g., via the safety protocol module 242) to the care asset management system 230.

The illustrative safety protocol management system 240 additionally includes a safety protocol module 242 that may be executed on one or more of the computing devices 104. The safety protocol module 242 may be embodied as, or otherwise include, any type of hardware, software, and/or firmware capable of performing the functions described herein. As will be described in further detail below, the safety protocol module 242 is configured to manage the safety protocols. To do so, the safety protocol module may be configured to retrieve the care asset state information of the care asset state database 214, the patient information of the patient information database 222, the care asset information of the care asset information database 232, and/or the care environment information of the care environment database 234.

Based on the retrieved data, the safety protocol module 242 can perform an analysis to determine whether a condition exists such that a care asset 120 or a current condition of a care asset 120 presents a health risk (e.g., a potentially adverse event) to a particular patient. For example, the safety protocol module 242 may analyze the retrieved data (e.g., care asset state information, patient information, care asset information, patient outcomes, etc.) and determine that a care asset 120 is a potential carrier of a health risk (e.g., a bacteria known to cause an infection) upon detecting that the care asset 120 came into direct or indirect contact with one or more patients whose outcomes resulted in development of the health risk (e.g., the infection). Similarly, the safety protocol module 242 can perform an analysis to determine whether a condition exists at a rate higher than a predicted rate, and identify a root cause (e.g., a care asset 120, a care activity, etc.). For example, a particular surface of a patient support 122 may have been used to support multiple patients, of which a higher than predicted rate experienced a particular health risk (e.g., a sacral pressure ulcer).

As a result of identifying a health risk, in use, the safety protocol module 242 can adjust the safety protocol (i.e., additional and/or alternative care assets 120 and/or alerts) based on the identified care asset 120 that has been determined to pose the health risk. For example, in the embodiment wherein use of the particular surface of a patient support 122 results in higher than the predicted rate as described above, the safety protocol module 242 may enact a particular protocol related to defective patient support 122 surface. In such embodiments, the safety protocol module 242 may reduce a maximum head-of-bed angle to 10%, reflecting the surface's inability to support the pelvic section, increase air flow to trade increased noise and decreased efficiency for optimized pressure management, and/or perform automated or manual local and/or remote communication the surface's compromised status, such as by warning a caregiver that the surface is not appropriate for high-risk patients or scheduling for maintenance, for example. It should be appreciated that any additional and/or alternative therapy may result based on the analysis. In other words, the safety protocol adjustment may be made based on a determination that a particular therapy is projected to result in an outcome that is less likely to introduce a health risk than another therapy.

In some embodiments, the safety protocol is adjusted by the detection of an action by a member of the hospital staff, such as the use of a particular care asset 120 and/or the setting of an alert. For example, in an embodiment wherein a patient is not identified as a falls risk patient, the activation of an exit detection system on a patient support apparatus 122 may cause the safety protocol module 242 to modify the patient's status to reflect that the patient has transitioned to become a falls risk. In another example, during compliance with the safety protocol, use of (e.g., opening, prepping, administering, etc.) a care asset 120 associated with the safety protocol may determine whether one or more additional care assets 120 associated with the safety protocol are assigned and/or whether one or more care activities associated with the safety protocol are scheduled, or being performed. In such embodiments, one or more care assets 120 relevant to the safety protocol may be sensed to determine whether the one or more care assets 120 relevant to the sub-protocol are in a desired state (e.g., assigned to the patient, in proximity to the patient, scheduled for delivery to the patient, at a desired position, etc.). For example, in an embodiment wherein a caregiver initiates a continuous lateral rotation therapy (CLRT) in compliance with a sub-protocol of the ventilator associated pneumonia (VAP) prevention protocol, a head-of-bed alarm may be set to sound if the head-of-bed angle is less than 30°, a sensory receivable message (e.g., "High infection risk - gloves and mask required!") may be initiated, and a respiratory therapy device automatically dispatched from a central supply to the patient's room, such as via the care asset management system 230.

Referring again to Fig. 1, the network 116 may be embodied as any type of wired or wireless communication network, including cellular networks (e.g., Global System for Mobile Communications (GSM), 3G, Long Term Evolution (LTE), Worldwide Interoperability for Microwave Access (WiMAX), etc.), digital subscriber line (DSL) networks, cable networks (e.g., coaxial networks, fiber networks, etc.), telephony networks, local area networks (LANs) or wide area networks (WANs), global networks (e.g., the Internet), or any combination thereof. As previously described, at least a portion the care assets 120 (e.g., the patient support apparatuses 122) may be in communication with the hospital information system 102 over the network 116. Accordingly, the network 116 may include any number of network devices (e.g., access points, routers, switches, servers, etc.) as needed to facilitate communications between the hospital information system 102 and the care assets 120 that are capable of network communication.

As noted previously, the care assets 120 include patient support apparatuses 122, as well as other care assets 126. The patient support apparatuses 122 may be embodied as, but are not limited to, a hospital bed, a chair, a toilet, a lift, a sling, and/or a stretcher. The other care assets 126 may include, but are not limited to, an article of clothing (e.g., gloves, socks, masks, gowns, etc.), a pad for a patient support apparatus 122 (e.g., a mattress pad for a hospital bed, a cushion for a chair, etc.), a bedding item for a patient support apparatus 122 (e.g., a sheet, a blanket, etc.), a catheter, a central line kit, and/or a ventilator. Still other care assets 126 may include monitors such as blood pressure monitors, EKG monitors, EEG monitors, SpO₂ monitors, heart rate monitors, respiration monitors, scales, thermometers and/or telemetry systems, etc. Still other care assets 126 may include therapy delivery systems including infusion systems, sequential compression devices, and/or exercise equipment, etc. While the illustrative healthcare communication system 100 includes a single patient support apparatus 122 contained within the structure 118, it should be appreciated that any number of patient support apparatuses 122 may be located in a healthcare communication system 100 of a healthcare facility. As such, any number of patient support apparatuses 122 may be communicatively coupled to the hospital information system 102 along with a combination of care assets 126.

As shown in Fig. 3, an illustrative embodiment of one of the patient support apparatuses 122 includes communication circuitry 300, a controller 302, and various sensors 312. The controller 302 is capable of controlling operational functionality of the patient support apparatus 122 and/or interpreting data signals from the various sensors 312. As noted previously, one or more of the other care assets 126 may be communicatively coupled to the hospital information system 102. Accordingly, those other care assets 126 capable of network communication may have similar electronic components to the patient support apparatuses 122 (although perhaps of different computation power, feature capability, and/or robustness).

Further, due to the differences associated with each of the patient support types (e.g., a hospital bed versus a chair), each of the patient support apparatuses 122 may include fewer, additional, or alternative electronic components to the illustrative patient support apparatus 122 of Fig. 3. In other words, certain features may not be pervasive across the patient support apparatuses 122 such that all of the components illustrated in Fig. 3 are representative of the components of every patient support apparatus 122. For example, some embodiments, such as a hospital bed embodiment, may be configured to facilitate network communication via wired and wireless technologies using additional or alternative protocols to those supported by another patient support apparatus 122 of the same or different type.

In another example, a hospital bed embodiment, a chair embodiment, or a toilet embodiment may have load cell sensors to detect a weight of a patient; however, the chair embodiment and/or the toilet embodiment likely do not have brakes, siderails, and/or adjustable height positions like that of the hospital bed embodiment. As such, the chair embodiment and the toilet embodiment would likely not include sensors and/or actuators typically associated with such features. Some of those similar components are shown in Fig. 3 and discussed below in regard to the illustrative patient support apparatus 122 with the understanding that such description is equally applicable to the similar components of other patient support apparatuses 122 and the other care assets 126 capable of network communication, with the understanding that certain differences may exist based on supported features.

The communication circuitry 300 is capable of establishing connections and facilitating communications to and from the patient support apparatus 122. The control system 124 is further configured to provide, or relay, status indications to a remote location, such as the nurse call system 210 of Fig. 2, via the communication circuitry 300. The status indications may include any type of indication of a component, or a patient relative to a component, of the patient support apparatus 122. The communication circuitry 300 may be embodied as any communication circuit, device, or collection thereof, capable of enabling communications over the network 116 between the patient support apparatus 122 and the hospital information system 102. The communication circuitry 300 may be configured to use any one or more communication technologies (e.g., wired or wireless communications) and associated protocols (e.g., Ethernet, Bluetooth®, Zigbee®, Wi-Fi®, WiMAX, etc.) to effect such communication.

The controller 302 of the control system 124 (i.e., an electronic control unit (ECU)) is connected to various sensors capable of being monitored and interpreted by the controller 302, and various actuators capable of being controlled by the controller 302. The controller 302 is configured to receive data (i.e., electrical signals) from the various sensors and components of the patient support apparatus 122, and control the operation of the components of the patient support apparatus 122 relative to the received data, as is known in the art. To do so, the controller 302 includes a number of electronic components commonly associated with controllers utilized in the control of electromechanical systems. For example, the controller 302 may include, amongst other components customarily included in such devices, a microprocessor 304 and a memory device 306. The memory device 306 may be, for example, a programmable read-only memory device ("PROM") including erasable PROM's (EPROM's or EEPROM's). In use, the memory device 306 is capable of storing, amongst other things, instructions in the form of, for example, a software routine (or routines) which, when executed by the microprocessor 304, allow the controller 302 to control operation of the features of the patient support apparatus 122.

The patient support apparatus 122 may include various settings for adjusting certain components of the patient support apparatus 122. For example, in some embodiments, such as in a hospital bed embodiment, the patient support apparatus 122 may include an actuator interface 308 and/or a pump interface 310, which the controller 302 may use to adjust certain components of the patient support apparatus 122. The patient support apparatus 122 may include a number of actuators (not shown) capable of being controlled by the control system 124 to control various actuator-based components (e.g., motors, valves, power control units, etc.) via the actuator interface 308. For example, a designated angle of a headrest portion of the patient support may be adjusted relative to a deck of the patient support via the actuator interface 308. Similarly, the patient support apparatus 122 may include a number of pumps (not shown) capable of being controlled by the control system 124 to control various pump-based components (e.g., fluid-housing mattress pads, hydraulics, etc.). For example, a designated pressure level of a component of the patient support apparatus 122 (e.g., a bladder of a mattress) may be controlled by the control system 124 via the pump interface 310. It should be appreciated that additional and/or alternative component interfaces may be present in other patient support apparatuses 122.

The sensors 312 may include various position sensors 314, actuator sensors 316, pressure sensors 318, and/or brake sensors 320, depending on the type of patient support apparatus 122 and the functionality supported by the patient support apparatus 122. The position sensors 314 may be configured to sense a position of a particular component of the patient support apparatus 122, such as a height of a deck of the patient support apparatus 122, a position of a of the patient support apparatus 122 (e.g., raised or lowered position), and/or a position of an upper frame of the patient support apparatus 122 (e.g., in a raised or lowered position relative to a base frame of the patient support apparatus 122).

The actuator sensors 316 may be configured to sense a state (e.g., open/closed, on/off, etc.) corresponding to each actuator component of the patient support. The pressure sensors 318 may be configured to sense a pressure (e.g., a fluid pressure) of a pump-based component of the patient support apparatus 122. The brake sensors 320 may be configured to sense whether brakes (e.g., casters) of the hospital bed embodiment are braked or released. It should be appreciated that the sensors 312 may include additional and/or alternative sensors, such as, but not limited to, motion sensors, temperature sensors, level sensors, other position sensors, etc.

Additionally, in some embodiments, the patient support apparatus 122 may include a user interface (UI) 322 for setting various alerts that are capable of alarming, such as through the nurse call system 210, based on detected events that correspond to the sensor data. The UI 322 may include a display, such as a touchscreen display capable of generating input data in response to being touched by the user. Accordingly, in some embodiments, the control system 124 may additionally include additional circuitry to convert between analog and digital signals, such as an analog-to-digital (A/D) converter (not shown) or the like. The UI 322 may include various input and output devices capable of receiving input from a user (e.g., a patient, hospital staff, caregiver, etc.) and/or providing output to the user related to various sensor and/or configuration data of the patient support apparatus 122.

Additionally or alternatively, in some embodiments, the UI 322 may include a light configuration and/or a speaker to provide status indications of the patient support to the patient and/or the hospital staff. Accordingly, in some embodiments, the UI 322 may include a keypad (e.g., a keyboard, a touchpad, etc.) for receiving user touch-based inputs and/or a microphone for receiving audible-based inputs. In some embodiments, one or more of the input and/or output devices may be mounted on a siderail of the hospital bed embodiment, connected to a control pendant of the hospital bed embodiment, etc. It should be appreciated that, in some embodiments, the UI 322 may additionally or alternatively include a communication port, or wireless access control panel, that may be accessed via a connected device (e.g., via universal serial bus (USB)) or an application running on a remote computing device in network communication (e.g., Bluetooth®) with the patient support apparatus 122.

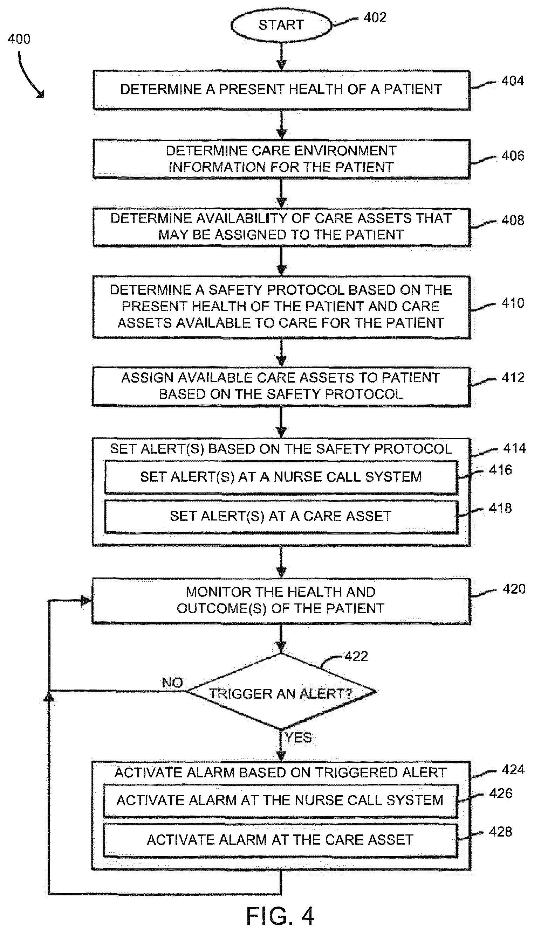

Referring now to Fig. 4, a process 400 for administering a safety protocol is shown that may be performed by one or more systems of the hospital information system 102 as shown in Fig. 2. The process 400 is initialized at step 402, which may be initiated by a patient triggered event, such as a patient intake event or an event that results in a change in a medical condition (i.e., health) of the patient, for example. The process 400 then proceeds to step 404, in which a present health of the patient is determined. The process 400 then proceeds to step 406, wherein care environment information for the patient is determined. As described previously, the care environment information includes any type of information indicative of the environment of a care asset 120 assigned to a patient, which may include, but is not limited to, a location, a temperature level, a lighting condition, a pressure reading, a position setting, an alert setting, etc. The process 100 then proceeds to step 408, wherein an availability state is determined for each of the care assets 120 that may be assigned to the patient. As described previously, the availability states may include an assigned state that indicates the care asset 120 is presently assigned to a patient, an available state that indicates the care asset 120 is in a condition such that it may be assigned to a patient, and an awaiting maintenance state that indicates the care asset 120 cannot be assigned until maintenance is performed on the care asset 120, for example.

The process 400 then proceeds to step 410, wherein a safety protocol is determined, such as by the safety protocol module 242 the safety protocol management system 240 of Fig. 2, based on the present health of the patient and the care assets 120 that are available to be assigned to the patient. As described previously, the safety protocol includes a number of various protocols, such as care protocols, therapy protocols, preventative protocols, etc., based on the present health conditions or expected conditions (i.e., a probable diagnosis) of a patient and/or the care assets 120 assigned to the patient. The safety protocol may be based on a patient scoring system used to determine a risk score using a set of established risk factors, for example.

As described previously, various sub-protocols of the safety protocol may be triggered by the care assets 120, such as by use and/or proximity to a patient or of a care asset 120 assigned to the patient. For example, if the patient is assigned a central line kit via the care asset management system 230, a central line infection prevention protocol may be activated upon detection of the central line kit being in proximity to the patient. Accordingly, other care assets 120 may be sensed or otherwise confirmed to be assigned, such as via the care asset management system 230, as directed by the set of defined procedures (i.e., instructions) associated with the safety protocol that, when executed, prompt staff members of the hospital to ensure certain precautions are taken. Further, additional or alternative alerts may be set to active, based on the activated protocol(s).

For example, upon detection of the central line kit, the triggered central line infection prevention protocol may provide (e.g., via the safety protocol module 242 of Fig. 2) an indication to the staff members that a gown, mask, skin cleaning agent, etc., must be present when the central line kit is used. Similarly, such secondary associated care assets 120 may be sensed in proximity to the patient, and, if not sensed, set to be dispensed from supply for a particular staff member assigned to the patient. In other embodiments, the precautions may include specifying a type of linen material to use for hospital bed linens for a patient with a skin condition, a minimum/maximum height threshold of a deck of the hospital bed, an angle threshold or angle range threshold of a headrest of the hospital bed, etc.

The process 400 then proceeds to step 412 wherein the safety protocol module 242 assigns the available care assets 120 identified in step 408 to the patient. To do so, for example, the safety protocol module 242 may provide an instruction to the care asset management system 230, which may further process the instruction and provide an indication to the hospital staff of which care assets 120 to be assigned to the patient. For example, in the example embodiment wherein the central line infection prevention protocol is implemented, a countdown alert for removal of the central line may be activated.

Further, the safety protocol includes a set of alerts that are to be set to ensure the safety of the patient. As such, the process 400 proceeds to step 414 to set one or more alerts based on the safety protocol. For example, in an embodiment wherein the patient has been identified as a falls risk, a falls prevention alert may be activated, or enabled. Activation of the falls prevention alert may further activate, or enable, a subset of alerts associated with the falls prevention alert, such as a toilet exit alert or a chair exit alert. To set, or activate, the alerts (i.e., to put the alerts into a monitoring state such that they can be alarmed if certain conditions are detected), at step 416, alerts can be set at a nurse call system, such as the nurse call system 210 of Fig. 2. Additionally or alternatively, at step 418, alerts may be set at a care asset 120, such as one of the patient support apparatuses 122, for example.

The process 400 further proceeds to step 420 to monitor the health and outcomes of the patient. From step 420, the process 400 proceeds to step 422 to determine whether an alert was triggered. In other words, to determine whether a condition was detected such that the alert is in an alarmed state. If not, the process 400 returns to step 420 to continue to monitor the health and outcomes of the patient. Otherwise, if an alert has been triggered into an alarmed state, the process 400 proceeds to step 424, where alarms, or notifications, associated with the triggered alerts are activated. To do so, at step 426, the alarms may be activated at the nurse call system 210. Additionally or alternatively, at step 428, the alarms may be activated at the care asset 120. From step 428, the process 400 returns to step 420 to continue to monitor the health and patient outcomes.

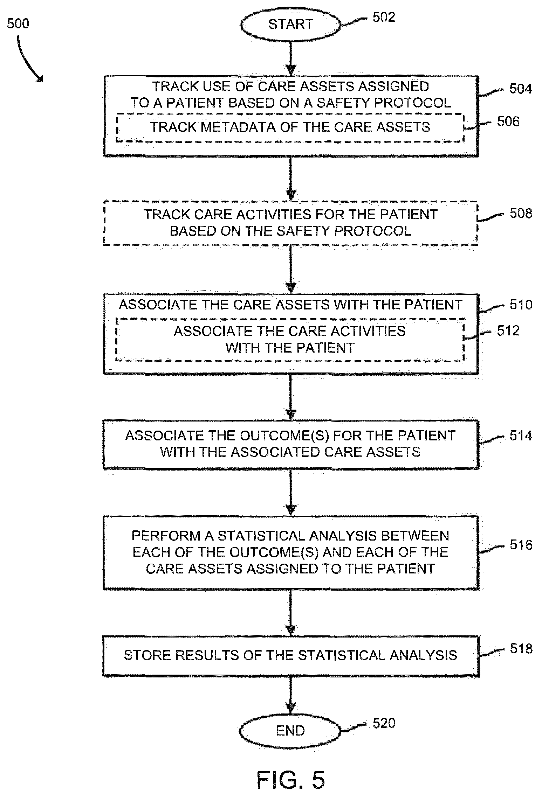

Referring now to Fig. 5, a process 500 for monitoring patient outcomes of an administered safety protocol that may be performed by one or more systems of the hospital information system 102, examples of which are illustratively shown in Fig. 2. The process 500 is initialized at step 502, which may be initiated by a safety protocol being implemented for a patient, such as at intake to the hospital or upon a change to the safety protocol. The process 500 proceeds to step 504, in which the usage of care assets 120 (e.g., one of the patient support apparatuses 122 and/or one or more of the other care assets 126) assigned to a patient, based on the safety protocol, are tracked. To do so, in some embodiments, metadata of the care assets 120 may be tracked at step 506. The metadata of the care assets 120 may include any data that identifies the care assets 120, such as a make identifier, a model identifier, a type identifier, a usage duration, a number of uses, a manufacturer identifier, and/or a manufactured date, for example.

In some embodiments, the process 500 may proceed from step 506 to step 508 to track care activities for the patient based on the safety protocol. The care activities may include any type of activity that is assigned to a staff member of the hospital based on the safety protocol. For example, a sub-protocol of a skin safety protocol may be a pressure ulcer prevention sub-protocol that may require a member of the hospital staff to regularly reposition the patient and/or perform a skin assessment, for example. In such embodiments, the care activities may be automatically tracked during rounding, or documented manually, such as by the hospital staff member in the EMR system 220 of Fig. 2.

The process 500 then proceeds to step 510 to associate the care assets assigned to the patient with the patient. It should be appreciated that, in some embodiments, this functionality may be performed automatically, such as by the care asset management system 230 of Fig. 2 or an ADT system. Additionally, at step 512, in some embodiments, the care activities tracked at step 508 may also be associated with the patient. The process 500 further proceeds to step 514 to associate outcomes of the patient with the care assets assigned to the patient that were tracked at step 504. To do so, each of the patient outcomes may be paired with the care assets assigned to that patient that contributed to a patient outcome. For example, whether a patient under the pressure ulcer prevention sub-protocol develop a pressure ulcer, or not, during their stay at the hospital. Additionally, the patient outcomes may include further information, such as, if the patient did develop a pressure ulcer, a stage of the pressure ulcer.

From step 514, the process 500 proceeds to step 516 to perform a statistical analysis between each of the patient outcomes and each of the care assets assigned to the patient. The statistical analysis may be used to determine whether a correlation and/or causation exist between the assigned care assets and the patient outcome. The process 500 then proceeds to step 518 to store the results of the statistical analysis before the process 500 proceeds to and terminates at step 520. In some embodiments, the results may be stored in the safety protocol database 244 of Fig. 2.

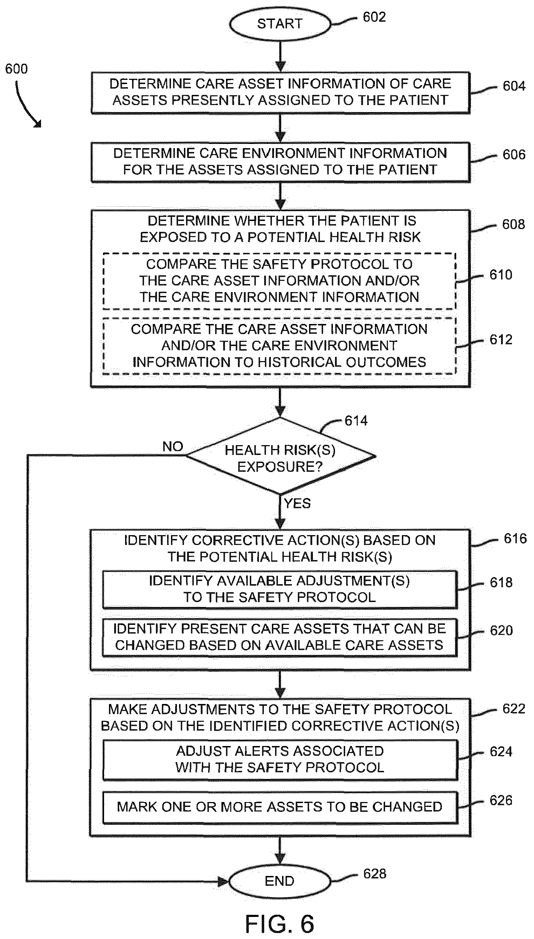

Referring now to Fig. 6, a process 600 for adjusting the administered safety protocol that may be performed by the safety protocol module 242 of the safety protocol management system 240 of Fig. 2. The process 600 is initialized at step 602, which may be initiated by a triggering event. The triggering event may correspond to a care asset 120 being utilized (e.g., opened, readied for use, etc.), a care asset 120 being assigned, or being scheduled to be assigned, to a particular patient, or a result of a statistical analysis (see Fig. 5) that indicates a health risk has been identified which may impact the patient and/or the safety protocol presently being deployed for the patient. In an example embodiment, wherein the care asset 120 is a central line kit, the triggering event may correspond to a detection that the central line kit was detected in proximity to the patient, or opened for use. In such an embodiment, a sensor or symbol attached to the central line kit may be scanned by a reader (e.g., a radio frequency identification (RFID) reader, a barcode scanner, etc.) such that an identifier that corresponds to the central line kit can be associated with the patient and/or a hospital room.

From step 602, the process proceeds to step 604, wherein the safety protocol module 242 determines care asset information for those care assets 120 that are presently assigned to the patient in accordance with the safety protocol. As described previously, the care asset information may include a component usage history, a maintenance history, an assignment history, an illness exposure history, and the like. At step 606, the safety protocol module 242 determines care environment information relative to the patient. As also described previously, the care environment information may include any type of information indicative of the environment of the care assets 120 assigned to a patient, such as a location, a temperature level, a lighting condition, a pressure reading, a position setting, an alert setting, etc. Accordingly, the safety protocol module 242 may determine a location of the patient based on information relative to one of the care assets 120, such as based on a weight sensor of a hospital bed or a tracking device embedded on a wristband worn by the patient, for example.

For example, in an embodiment, the safety protocol module 242 may determine that the patient is presently under a particular prevention sub-protocol, is located in a particular room, and is being cared for with a particular model of hospital bed with a certain type of mattress on the hospital bed. It should be appreciated that, in some embodiments, the care environment information and/or the care asset information may be automatically transmitted to the safety protocol module 242; while, in other embodiments, the care environment information and/or the care asset information may be retrieved by the safety protocol module 242.

The process 600 proceeds to step 608, wherein the safety protocol module 242 determines whether the patient is exposed to a potential health risk. To do so, in some embodiments, at step 610, the safety protocol module 242 may compare the present safety protocol to the care asset information and/or the care environment information. Additionally or alternatively, in some embodiments, at step 612, the safety protocol module 242 may compare the care asset information and the care environment information to historical outcome information, such as the results of the statistical analysis performed by the safety protocol module 242 (see Fig. 5). In other words, the safety protocol module 242 may determine whether the care assets 120 assigned to a previous patient may have directly contributed to a medical condition exhibited by that previous patient, and whether the patient presently assigned the care assets 120 is at risk of experiencing the same outcome.

The process 600 then proceeds to step 614 to determine whether the safety protocol module 242 has determined that the patient is exposed to a potential health risk. For example, in furtherance of the example embodiment with the patient under a pressure ulcer prevention protocol as described above, the patient may be compared to any number of other patients susceptible to pressure ulcers who also used the same model of hospital bed and/or the same type of mattress or sheets on the hospital bed. Accordingly, a percentage of the other patients that developed a pressure ulcer may be determined and compared to a threshold percentage. In such an embodiment, if the percentage of the other patients that developed a pressure ulcer exceeds the threshold percentage, the safety protocol module 242 may determine that the patient is exposed to a potential health risk.

Further, the safety protocol module 242 may determine a relationship between the patients that developed a pressure ulcer and the model of hospital bed, type of mattress, and/or type of sheet. In such an embodiment, the safety protocol module 242 may determine what woven sheets were assigned to a majority of the patients that developed a pressure ulcer. Accordingly, as will be discussed in further detail below, the woven sheets may be removed, or replaced with knit sheets, for those patients with an active skin care protocol to address pressure ulcers. In other words, the pressure ulcer prevention sub-protocol may be changed from assigning woven sheets to knit sheets and the existing pressure ulcer prevention sub-protocol patients that are assigned woven sheets scheduled to receive knit sheets, for example.

In another example, the safety protocol module 242 may determine the present safety protocol to be insufficient based on the comparison performed at block 610. In such an embodiment, the presence and/or use of (e.g., opening, prepping, administering, etc.) a care asset 120 may trigger one or more additional and/or alternative sub-protocols to be activated, thereby rendering the present safety protocol insufficient. For example, if a patient is determined to be in need of a lift after an existing safety protocol was enacted, one or more sub-protocols corresponding to care of the patient with respect to the lift may be activated. Similarly, for example, in an embodiment wherein a patient has not been identified as a falls risk and a member of the hospital staff sets an exit alarm for a patient support 122 (e.g., a hospital bed), one or more additional and/or alternative sub-protocols, such as the falls risk protocol, may be activated. Accordingly, as will be described in further detail below, alerts and/or care assets 120 may be adjusted based on the additional and/or alternative sub-protocols.

It should be appreciated that additional and/or alternative data may be used to further refine the results. In other words, the percentage of the other patients that developed a pressure ulcer may be further restricted by additional information consistent with the care assets presently assigned to the patient. For example, the percentage of the other patients that developed a pressure ulcer may increase if a portion of the other patients are removed based on one or more criteria, such as an age of the hospital bed or a number of uses of the mattress. Accordingly, the refined percentage may provide a more accurate representation of the health risk exposure. Similarly, certain alerts and/or care activities that were tracked for previous patients may influence the determined health risk exposure.

If the safety protocol module 242 has determined that the patient is not exposed to a health risk, the process 600 proceeds to step 628, wherein the process 600 terminates. Otherwise, if the safety protocol module 242 has determined that the patient is exposed to a potential health risk, the process 600 advances to step 616 to identify corrective actions based on the potential health risk exposure. To do so, in step 618, the safety protocol module 242 identifies available adjustments that can be made to the safety protocol, such as by changing alerts, care activities, a security level, etc., of the safety protocol. Additionally, in step 620, the safety protocol module 242 identifies which of the care assets 120 presently assigned to the patient can be changed based on the health risk and the care assets 120 that are available to be assigned to the patient. For example, the statistical analysis of the patient outcomes may determine a particular care asset 120 was assigned to several different patients, a percentage of which contracted an infection after coming into direct or indirect contact with that particular care asset 120.

From step 620, the process 600 proceeds to step 622 to make adjustments to the safety protocol based on the identified corrective actions at step 616. At block 624, the safety protocol module 242 adjusts the safety protocol based on the adjustments identified at step 618. To do so, the safety protocol module 242 may adjust the alerts at the nurse call system 210 and/or one or more of the care assets 120 that are capable of supporting alerts. It should be appreciated that the adjustments may be implemented manually or automatically, depending on the supported functionality of the care assets 120 affected by the adjustment to the safety protocol.

Additionally, at block 626, the safety protocol module 242 marks one or more assets to be changed that were identified at step 620. To do so, the safety protocol module 242 may provide an instruction to the care asset management system 230 that indicates that one or more care assets 120 to be changed. The process 600 then proceeds to step 628, wherein the process 600 terminates. Referring again to the particular care asset 120 that was assigned to several different patients, a percentage of which contracted an infection, may be marked as "out of service," or otherwise scheduled for maintenance. Further, in some embodiments, the particular care asset 120 may be disabled, if the particular care asset 120 supports such a feature, such as in an embodiment wherein the particular care asset 120 is a pump that is not presently in use.

Although certain illustrative embodiments have been described in detail above, variations and modifications exist.

## Claims

1. A system comprising:
a patient support apparatus including a plurality of sensors coupled to a communication network,
a user input device coupled to the communication network, and
a safety protocol management system coupled to the communication network, the safety protocol management system including a patient safety protocol module, wherein the safety protocol management system is configured to receive information from the patient support apparatus and user input device over the communication network and to manage a patient safety protocol for a patient associated with the patient support apparatus to address a safety risk of the patient, and wherein each of the patient safety protocols is based on health information of the patient,
wherein the patient safety protocol module is configured to retrieve historical outcomes for other patients that were associated with the patient support apparatus, identify the patient support apparatus as a root cause of a negative patient outcome for the other patients, and adjust a patient safety protocol based on the root cause determination.

2. The system of claim 1, wherein the patient support apparatus communicates a change in an operating parameter of the patient support apparatus over the communication network and the a safety protocol management system monitors the communication network for changes in parameters of the patient support apparatus to determine a safety risk of the patient.

3. The system of claim 2, wherein a change in an operating parameter of the patient support apparatus is processed by the safety protocol management system to update a risk profile of the patient on the patient support apparatus.

4. The system of claim 3, wherein the system further comprises an electronic medical records system, wherein an input to the user input device indicative of a desired change in an operating parameter of the patient support apparatus is communicated over the communication network, and wherein the input is processed by the electronic medical records system to update the patient's electronic medical record to indicate that the patient has developed a risk factor associated with the change in the changed operating parameter.

5. The patient support apparatus of claim 4, wherein the safety protocol module processes the risk factor associated with the change in operating parameter to re-evaluate the risk of an adverse event to the patient, and, if the risk of an adverse event meets a predetermined threshold, alert a caregiver of the risk.

6. The system of any preceding claim, wherein adjusting the patient safety protocol includes identifying an environmental condition that should be modified to reduce the risk of an adverse event occurring for the patient and alerting a caregiver to modify the environmental condition.

7. The system of any preceding claim, wherein adjusting the patient safety protocol includes identifying a parameter of the patient support apparats that should be modified to reduce the risk of an adverse event occurring for the patient and alerting a caregiver to modify the parameter.

8. The system of any preceding claim, wherein adjusting the patient safety protocol includes identifying an alternate patient support apparatus that should be used for the patient and alerting a caregiver to substitute the alternate patient support apparatus.

9. The system of claim 8, wherein the system further comprises an electronic medical records system coupled to the communication network, wherein the identifying an alternate patient support apparatus includes considering details of the patient's medical record to determine a risk profile of the patient and utilize the risk profile to identify an appropriate alternate patient support apparatus to reduce the risk profile.

10. The system of claim 9, wherein the system further comprises an asset management system for monitoring the availability of patient support apparatuses, wherein the identifying an alternate patient support apparatus includes considering the availability of alternate patient support apparatuses to identify an appropriate alternate patient support apparatus.

11. The system of claim 10, wherein adjusting the patient safety protocol includes identifying an optimal parameter setting of the alternate patient support apparats that should be selected to reduce the risk of an adverse event occurring for the patient and alerting a caregiver to select the optimal parameter on the alternate patient support apparatus.

12. The system of claim 11, wherein adjusting the patient safety protocol includes identifying an optimal environmental setting that should be selected to reduce the risk of an adverse event occurring for the patient and alerting a caregiver to select the optimal environmental setting.

13. The system of any of any preceding claim, wherein an operating parameter of the patient support apparatus includes head angle, an alarm setting, a therapy setting, a component orientation, a mattress pressure, a brake setting, a siderail position, or a motion status.

14. The system of any preceding claim, wherein an environmental condition includes a location, a temperature level, a lighting condition, a pressure reading, a position setting, or an alert setting.

15. The system of any preceding claim, wherein the user input device receives information related to the use of a care asset such as an alternative patient support, a chair, a toilet, a lift, a sling, a stretcher, an article of clothing, gloves, socks, masks, gowns, a pad, a bedding item, a sheet, a blanket, a catheter, a central line kit, a ventilator, a blood pressure monitor, an EKG monitor, an EEG monitor, a SpO₂ monitor, a heart rate monitor, a respiration monitor, a scale, a thermometer, a telemetry system, an infusion system, a sequential
